# EUROPEAN PATENT APPLICATION

(11) **EP 4 851 279 A1**
(43) Date of publication of application: **09.12.2026**
(21) Application number: 25748940.1
(22) Date of filing: 31.01.2025
(51) Int. Cl.: C12N 1/20, A23C 9/123, A23L 33/135, C12N 1/21, C12N 15/31, C12P 1/04, C12Q 1/04, C12Q 1/689

(54) **OXYGEN-TOLERANT BACTERIUM OF GENUS BIFIDOBACTERIUM, CULTURE METHOD AND SCREENING METHOD FOR SAID BACTERIUM, COMPOSITION CONTAINING OXYGEN-TOLERANT BACTERIUM OF GENUS BIFIDOBACTERIUM, AND METHOD FOR PRODUCING SAID COMPOSITION**

(30) Priority: 31.01.2024 JP 2024012774
(71) Applicant: Morinaga Milk Industry Co., Ltd., Minato-ku Tokyo 105-7122 (JP)
(72) Inventor: KANEKO, Hiroki, Zama-shi, Kanagawa 252-8583 (JP); ODAMAKI, Toshitaka, Zama-shi, Kanagawa 252-8583 (JP)
(74) Representative: Vossius & Partner Patentanwälte Rechtsanwälte mbB
(86) International application number: PCT/JP2025/003173
(87) International publication number: WO 2025/164761

(57) **Abstract**

A problem of the invention is to provide a bacterium of the genus *Bifidobacterium* having oxygen tolerance which can be cultured under aerobic conditions, a culture method thereof and a screening method thereof as well as a composition containing a bacterium of the genus *Bifidobacterium* having oxygen tolerance and a production method thereof. The invention, which solves the problem, is a method for culturing a bacterium of the genus *Bifidobacterium* having any one gene selected from (1) to (7) below including a step of culturing the bacterium of the genus *Bifidobacterium* under aerobic conditions. (1) A gene consisting of a base sequence represented by SEQ ID NO: 1 or 2. (2) A gene having an identity of 90% or more with the base sequence represented by SEQ ID NO: 1 or 2. (3) A gene consisting of the base sequence represented by SEQ ID NO: 1 or 2 in which one to several bases have been deleted, substituted or added. (4) A gene consisting of a base sequence of DNA which hybridizes to DNA consisting of a complementary sequence of the base sequence represented by SEQ ID NO: 1 or 2 under stringent conditions. (5) A gene consisting of a degenerate variant of the base sequence represented by SEQ ID NO: 1 or 2. (6) A gene encoding a protein consisting of an amino acid sequence represented by SEQ ID NO: 3 or 4. (7) A gene encoding a protein consisting of the amino acid sequence represented by SEQ ID NO: 3 or 4 in which one to several amino acids have been deleted, substituted or added.

## Description

### Technical Field

The present invention relates to a bacterium of the genus *Bifidobacterium* having oxygen tolerance, a culture method thereof and a screening method thereof as well as a composition containing a bacterium of the genus *Bifidobacterium* having oxygen tolerance and a production method thereof.

### Background Art

Bacteria of the genus *Bifidobacterium* (so-called bifidobacteria), which are one type of intestinal bacteria, have been found to have various actions which help the health of humans and the like and are used for foods, drinks and medicines. There are a huge number of medicines and foods in which bifidobacteria are used, and bifidobacteria are often used especially for dairy products such as fermented milk (yogurt). Bifidobacteria, however, are obligate anaerobes which require anaerobic conditions for the survival, and thus it is difficult to handle bifidobacteria in an aerobic environment.

While there has been such a problem, a bacterial strain which excellently grows aerobically has been isolated from feces of a healthy breastfed infant (*Bifidobacterium breve* SBR3212 (the Fermentation Research Institute, bacterial deposit No. 11915)), and it has been proposed to use the strain as a starter for a fermented milk (PTL 1).

### Citation List

### Patent Literature

PTL 1: JPH04-320642A

### Non Patent Literature

NPL 1: Salvatore Cosentino, Wataru Iwasaki, "SonicParanoid2: fast, accurate, and comprehensive orthology inference with machine learning and language models", bioRxiv 2023.05.14.540736; doi: https://doi.org/10.1101/2023.05.14.540736
NPL 2: Yu T, Cui H, Li JC, Luo Y, Jiang G, Zhao H., "Enzyme function prediction using contrastive learning.", Science. 2023 Mar 31; 379(6639): 1358-1363.doi: 10.1126/science.adf2465.Epub 2023 Mar 30., PMID: 36996195
NPL 3: Kozakai T, Nakajima A, Miyazawa K, Sasaki Y, Odamaki T, Katoh T, Fukuma T, Xiao JZ, Suzuki T, Katayama T, Sakanaka M. "An improved temperature-sensitive shuttle vector system for scarless gene deletion in human-gut-associated Bifidobacterium species.", iScience. 2024 Oct 1; 27(11): 111080.doi: 10.1016/j.isci.2024.111080.PMID: 39502284; PMCID: PMC11536034.

### Summary of Invention

### Technical Problem

PTL 1 discloses a bacterium of the genus *Bifidobacterium* having oxygen tolerance which can be cultured under aerobic conditions. It has been unknown, however, which factor the bacterium of the genus *Bifidobacterium*, which is originally known to be an obligate anaerobe, has to exhibit oxygen tolerance.

Considering such an issue, a problem of the invention is to provide a bacterium of the genus *Bifidobacterium* having oxygen tolerance, a culture method thereof and a screening method thereof as well as a composition containing a bacterium of the genus *Bifidobacterium* having oxygen tolerance and a production method thereof.

### Solution to Problem

After intensive studies and efforts, the present inventors have found that a bacterium of the genus *Bifidobacterium* having a gene consisting of a base sequence represented by SEQ ID NO: 1 or 2 has oxygen tolerance and thus have completed the invention. The invention which solves the problem is as follows.
[1] A method for culturing a bacterium of the genus *Bifidobacterium* having any one gene selected from (1) to (7) below, including a step of culturing the bacterium of the genus *Bifidobacterium* under aerobic conditions.
   (1) A gene consisting of a base sequence represented by SEQ ID NO: 1 or 2.
   (2) A gene having an identity of 90% or more with the base sequence represented by SEQ ID NO: 1 or 2.
   (3) A gene consisting of the base sequence represented by SEQ ID NO: 1 or 2 in which one to several bases have been deleted, substituted or added.
   (4) A gene consisting of a base sequence of DNA which hybridizes to DNA consisting of a complementary sequence of the base sequence represented by SEQ ID NO: 1 or 2 under stringent conditions.
   (5) A gene consisting of a degenerate variant of the base sequence represented by SEQ ID NO: 1 or 2.
   (6) A gene encoding a protein consisting of an amino acid sequence represented by SEQ ID NO: 3 or 4.
   (7) A gene encoding a protein consisting of the amino acid sequence represented by SEQ ID NO: 3 or 4 in which one to several amino acids have been deleted, substituted or added.
[2] The culture method according to [1] in which the bacterium of the genus *Bifidobacterium* is *Bifidobacterium longum*.
[3] The culture method according to [1] in which the bacterium of the genus *Bifidobacterium* is *Bifidobacterium longum* subsp. *infantis*.
[4] The culture method according to [1] in which the bacterium of the genus *Bifidobacterium* is *Bifidobacterium longum* subsp. *infantis* MCC02042 (NITE BP-03068).
[5] A method for producing a composition containing a bacterium of the genus *Bifidobacterium* including the culture method according to any one of [1] to [4].
[6] The production method according to [5] including a step of drying a bacterial culture containing the bacterium of the genus *Bifidobacterium* cultured by the culture method
   in which the composition is a bacterial powder obtained by the drying or a food composition containing the bacterial powder.
[7] The production method according to [5] including a step of adding a starter culture containing the bacterium of the genus *Bifidobacterium* which has been cultured by the culture method to a raw material composition and fermenting.
[8] A modified bacterium of the genus *Bifidobacterium* having enhanced or conferred oxygen tolerance obtained by introducing any one gene selected from (1) to (7) above.
[9] A method for screening a bacterium of the genus *Bifidobacterium* having oxygen tolerance including a step of selecting a bacterium of the genus *Bifidobacterium* using any one gene selected from (1) to (7) above as an indicator.
[10] A composition for a starter culture containing a bacterium of the genus *Bifidobacterium* having any one gene selected from (1) to (7) above.
[11] A composition containing
   a bacterium of the genus *Bifidobacterium* having any one gene selected from (1) to (7) above and
   one, two or more lactic acid bacteria selected from *Lactococcus lactis*, *Streptococcus thermophilus*, *Lactobacillus bulgaricus* and *Lactobacillus delbrueckii*.

### Advantageous Effects of Invention

According to the invention, a bacterium of the genus *Bifidobacterium* having oxygen tolerance, a culture method thereof and a screening method thereof as well as a composition containing the bacterium of the genus *Bifidobacterium* and a production method thereof can be provided.

### Description of Embodiments

Preferable embodiments of the invention will be explained. However, the invention is not limited to the following preferable embodiments and can be changed freely in the scope of the invention. In the present specification, the percentages are based on mass unless otherwise specified.

### <Culture Method>

The culture method of the invention is characterized by including a step of culturing a bacterium of the genus *Bifidobacterium* having any one gene selected from (1) to (7) below under aerobic conditions.

Here, (1) the gene consisting of a base sequence represented by SEQ ID NO: 1 or 2 has been found through the comparative genomics in the test example described later.
(1) A gene consisting of a base sequence represented by SEQ ID NO: 1 or 2.
(2) A gene having an identity of 90% or more with the base sequence represented by SEQ ID NO: 1 or 2.
(3) A gene consisting of the base sequence represented by SEQ ID NO: 1 or 2 in which one to several bases have been deleted, substituted or added.
(4) A gene consisting of a base sequence of DNA which hybridizes to DNA consisting of a complementary sequence of the base sequence represented by SEQ ID NO: 1 or 2 under stringent conditions.
(5) A gene consisting of a degenerate variant of the base sequence represented by SEQ ID NO: 1 or 2.
(6) A gene encoding a protein consisting of an amino acid sequence represented by SEQ ID NO: 3 or 4.
(7) A gene encoding a protein consisting of the amino acid sequence represented by SEQ ID NO: 3 or 4 in which one to several amino acids have been deleted, substituted or added.

Here, the base sequences represented by SEQ ID NO: 1 and SEQ ID NO: 2 and the amino acid sequences represented by SEQ ID NOs: 3 and 4 are as shown in the table below.

**[Table 1]**

| | |
|---|---|
| SEQ ID NO: 1 | atgaatattttagttacgggtggacttgggtacaccggaattgaactagtaaaaa actaagtaatactggtcataatgttgtcatagtagatatagacttgtatgatacgg cgcgtttcttggaaagctgccctaatgttaagattctcaaagctgatatcttgacg gtcgattggtctgttattttacgacagtattcaatcgatgtggtctatcacctagc ggggatttcgaacgatcctggtaatggtgtgacagctgagtttggtatgaaaatca attattatgcatccgtgcgcctttatgaaagtcttagattcagtttggtgcataaa ttcatttatccttcttcctgtagcgtatatggactctctcaaaacaacgcagtaga tgagacgtgcccaatttcaccaattacaaactatgcacggtgcaaagtaatggttg agcaatacgtgcgtcaacatcaatatccaaacatttcaacagtcttattgcgtcct gctacggtgttcggcccctcaactagacagcgtctcgatttgctcataaaccgtac tattatcggcgcgctagcgggaaccgctattcatacacctaaccttgaaaacagca gacctcatgtatatatccaagatttgttgcgggtctatgaattcttcttaacccat ggtggcgggcttaatacatataatatcgcttttggtaattataaaatacgagatat agttagaaaggtccaattagtaacaggtaaaagtattcacatatgtgaagacaaaa aagcagatccacggacatattgcgtaaattcgaagagaattctatctgttttccct gatctttcgaaagcttccttcgaagaaaatctttccgccacagttacttcatttat aggacaagatcttatcgcaaaaatcgatgaagatatttactataacaagagaattc aagaaaagaagtgggggacatctctgtga |
| SEQ ID NO: 2 | atgctcgctaaccccagcgaagtcccggaatggtcgactgcagatgtcgcaagaca atccggcacttcccccgccacagtggtacgtgcatgtcagcgcattggtttccgcg gttatcagcatctacgactggaattcgctcgtctgactgaaacttcgcaggaagac atgaccgctaatgaagatgacgacgtgtccacggcgatggcggtgttccatgacgc agtcgaggcattgcggctcacaacgagatcgctggatcaggttgccctccttcatg |
| | ccacagagatagtgtcgtctgccggtcgtgtgctatgtacagccagcggattctcg ctacccccctctgcaggacgccgcgttacgactaaccacatcggggattgatgtatc ggctccgcaggatgtcattgcccagcaatttggagcgcattccctaggcccgcaag atgtatgtctcgcagtaagctattctggggcgaatacacacactttgcgaacctgc caaatcgccaagcaacagggtgccagtgtgattgccgtaacaaacttctctaaatc gccgctggccgatctctcggatgtattactggtcaccggcgtcgcaccggttcgcc acagtgttgacccatccgctgaccgcgtggcgcaaatgtgtatgttgcatgcccta tcgacgctagtcgcccaccgctgcaccgataccacacccgacatgcgacaggtggt ggccgattccattagcgaacatcttgaccagtga |
| SEQ ID NO: 3 | MNILVTGGLGYTGIELVKKLSNTGHNVVIVDIDLYDTARFLESCPNVKILKADILT VDWSVILRQYSIDVVYHLAGISNDPGNGVTAEFGMKINYYASVRLYESLRFSLVHK FIYPSSCSVYGLSQNNAVDETCPISPITNYARCKVMVEQYVRQHQYPNISTVLLRP ATVFGPSTRQRLDLLINRTIIGALAGTAIHTPNLENSRPHVYIQDLLRVYEFFLTH GGGLNTYNIAFGNYKIRDIVRKVQLVTGKSIHICEDKKADPRTYCVNSKRILSVFP DLSKASFEENLSATVTSFIGQDLIAKIDEDIYYNKRIQEKKWGTSL |
| SEQ ID NO: 4 | MLANPSEVPEWSTADVARQSGTSPATVVRACQRIGFRGYQHLRLEFARLTETSQED MTANEDDDVSTAMAVFHDAVEALRLTTRSLDQVALLHATEIVSSAGRVLCTASGFS LPPLQDAALRLTTSGIDVSAPQDVIAQQFGAHSLGPQDVCLAVSYSGANTHTLRTC QIAKQQGASVIAVTNFSKSPLADLSDVLLVTGVAPVRHSVDPSADRVAQMCMLHAL STLVAHRCTDTTPDMRQVVADSISEHLDQ |

The bacterium of the genus *Bifidobacterium* having any one gene selected from (1) to (7) has oxygen tolerance and thus can be cultured under aerobic conditions.

For convenience of explanation, "any one gene selected from (1) to (7)" is sometimes referred to as "the gene of this case" below. Moreover, the "bacterium of the genus *Bifidobacterium* having any one gene selected from (1) to (7)" is sometimes referred to as "the bacterium of the genus *Bifidobacterium* of this case".

The bacterium of the genus *Bifidobacterium* of this case may be a bacterium acquired from an animal including a human or environment. Moreover, the bacterium of the genus *Bifidobacterium* of this case may be a bacterium artificially produced by introducing the gene of this case by a genetic engineering method.

The bacterium of the genus *Bifidobacterium* of this case may be a bacterial cell line having the identical genetic background. Moreover, the bacterium of the genus *Bifidobacterium* of this case may be a population of cells which do not have an identical genetic background as long as the cells have the gene of this case in common.

The bacterium of the genus *Bifidobacterium* of this case is not particularly limited, but examples thereof include *Bifidobacterium longum*, *Bifidobacterium breve*, *Bifidobacterium bifidum*, *Bifidobacterium adolescentis*, *Bifidobacterium animalis*, *Bifidobacterium pseudolongum*, *Bifidobacterium reuteri*, *Bifidobacterium catenulatum*, *Bifidobacterium pseudocatenulatum* and *Bifidobacterium lactis*.

In a preferable embodiment of the invention, the bacterium of the genus *Bifidobacterium* of this case is *Bifidobacterium longum*.

More specifically, the bacterium of the genus *Bifidobacterium* of this case is preferably *Bifidobacterium longum* subsp. *infantis*.

Further specifically, the bacterium of the genus *Bifidobacterium* of this case is preferably *Bifidobacterium longum* subsp. *infantis* MCC02042 (NITE BP-03068).

In an embodiment, the bacterium of the genus *Bifidobacterium* of this case does not include *Bifidobacterium breve* SBR3212 (the Fermentation Research Institute, bacterial deposit No. 11915).

### [Gene]

### (1) Gene Consisting of Base Sequence Represented by SEQ ID NO: 1 or 2

The gene consisting of the base sequence represented by SEQ ID NO: 1 is a gene encoding SDR (NADH oxidoreductase).

The gene consisting of the base sequence represented by SEQ ID NO: 2 is a gene encoding transcriptional regulator.

The two genes above are genes commonly possessed by bacterial cell lines of the genus *Bifidobacterium* having oxygen tolerance which can be cultured under aerobic conditions and are genes which have been concluded to be involved in oxygen tolerance. Thus, a bacterium of the genus *Bifidobacterium* having the gene consisting of the base sequence represented by SEQ ID NO: 1 or 2 has oxygen tolerance and can be cultured under aerobic conditions.

The bacterium of the genus *Bifidobacterium* of this case may be in a form having either one of the genes below or a form having both thereof,
a gene consisting of the base sequence represented by SEQ ID NO: 1 and
a gene consisting of the base sequence represented by SEQ ID NO: 2.

### (2) Gene Having Identity of 90% or More with Base Sequence Represented by SEQ ID NO: 1 or 2

The bacterium of the genus *Bifidobacterium* of this case may be in a form having either one of the genes below or a form having both thereof,
a gene having an identity of 90% or more with the base sequence represented by SEQ ID NO: 1 and
a gene having an identity of 90% or more with the base sequence represented by SEQ ID NO: 2.

In the gene of (2) above, the identity is preferably 93% or more, more preferably 95% or more, more preferably 98% or more, further preferably 99% or more.

The gene of (2) above is preferably a gene which has an identity of 90% or more with the base sequence represented by SEQ ID NO: 1 or 2 and which has a function of enhancing or conferring oxygen tolerance when the gene is introduced to a bacterium of the genus *Bifidobacterium*.

### (3) Gene Consisting of Base Sequence Represented by SEQ ID NO: 1 or 2 in Which One to Several Bases Have Been Deleted, Substituted or Added

The bacterium of the genus *Bifidobacterium* of this case may be in a form having either one of the genes below or a form having both thereof,
a gene consisting of the base sequence represented by SEQ ID NO: 1 in which one to several bases have been deleted, substituted or added and
a gene consisting of the base sequence represented by SEQ ID NO: 2 in which one to several bases have been deleted, substituted or added.

In the gene of (3) above, the number of the bases which have been deleted, substituted or added is preferably one to 100, more preferably one to 90, further preferably one to 70, further preferably one to 50, further preferably one to 20, further preferably one to 10, further preferably one to five.

The gene of (3) above is preferably a gene that consists of the base sequence represented by SEQ ID NO: 1 or 2 in which one to several bases have been deleted, substituted or added and that has a function of enhancing or conferring oxygen tolerance when the gene is introduced to a bacterium of the genus *Bifidobacterium*.

### (4) Gene Consisting of Base Sequence of DNA Which Hybridizes to DNA Consisting of Complementary Sequence of Base Sequence Represented by SEQ ID NO: 1 or 2 under Stringent Condition

The bacterium of the genus *Bifidobacterium* of this case may be in a form having either one of the genes below or a form having both thereof,
a gene consisting of a base sequence of DNA which hybridizes to DNA consisting of a complementary sequence of the base sequence represented by SEQ ID NO: 1 under stringent conditions and
a gene consisting of a base sequence of DNA which hybridizes to DNA consisting of a complementary sequence of the base sequence represented by SEQ ID NO: 2 under stringent conditions.

The expression "under stringent conditions" in the present specification means that the DNA hybridizes under moderately or highly stringent conditions. Specifically, the moderately stringent conditions can be easily decided by one skilled in the art having a general skill, for example, based on the length of the DNA. Basic conditions are shown in Sambrook et al, Molecular Cloning: A Laboratory Manual, third edition, sections 6-7, Cold Spring Harbor Laboratory Press, 2001. Preferably, the moderately stringent conditions in terms of the hybridization conditions are conditions of 1× SSC to 6× SSC and 42°C to 55°C, more preferably conditions of 1× SSC to 3× SSC and 45°C to 50°C, most preferably conditions of 2× SSC and 50°C. When about 50% formamide, for example, is contained in the hybridization solution, a temperature that is 5 to 15°C lower than the above temperature is used. Washing conditions include 0.5× SSC to 6× SSC and 40°C to 60°C. During the hybridization and the washing, 0.05% to 0.2%, preferably about 0.1% SDS may be generally added. The highly stringent conditions can also be easily decided by one skilled in the art, for example, based on the length of the DNA. In general, the highly stringent (high-stringent) conditions include hybridization and/or washing at a higher temperature and/or a lower salt concentration than those of the moderately stringent conditions. Examples of the hybridization conditions include conditions of 0.1× SSC to 2× SSC and 55°C to 65°C, more preferably conditions of 0.1× SSC to 1× SSC and 60°C to 65°C, most preferably conditions of 0.2× SSC and 63°C. The washing conditions include 0.2× SSC to 2× SSC and 50°C to 68°C, more preferably 0.2× SSC and 60 to 65°C.

The gene of (4) above is preferably a gene that consists of a base sequence of DNA which hybridizes to DNA consisting of a complementary sequence of the base sequence represented by SEQ ID NO: 1 or 2 under stringent conditions and that has a function of enhancing or conferring oxygen tolerance when the gene is introduced to a bacterium of the genus *Bifidobacterium*.

### (5) Gene Consisting of Degenerate Variant of Base Sequence Represented by SEQ ID NO: 1 or 2

The bacterium of the genus *Bifidobacterium* of this case may be in a form having either one of the genes below or a form having both thereof,
a gene consisting of a degenerate variant of the base sequence represented by SEQ ID NO: 1 and
a gene consisting of a degenerate variant of the base sequence represented by SEQ ID NO: 2.

### (6) Gene Encoding Protein Consisting of Amino Acid Sequence Represented by SEQ ID NO: 3 or 4

The protein consisting of the amino acid sequence represented by SEQ ID NO: 3 is SDR (NADH oxidoreductase).

The protein consisting of the amino acid sequence represented by SEQ ID NO: 4 is Transcriptional regulator.

The bacterium of the genus *Bifidobacterium* of this case may be in a form having either one of the genes below or a form having both thereof,
a gene encoding a protein consisting of the amino acid sequence represented by SEQ ID NO: 3 and
a gene encoding a protein consisting of the amino acid sequence represented by SEQ ID NO: 4.

### (7) Gene Encoding Protein Consisting of Amino Acid Sequence Represented by SEQ ID NO: 3 or 4 in Which One to Several Amino Acids Have Been Deleted, Substituted or Added

The bacterium of the genus *Bifidobacterium* of this case may be in a form having either one of the genes below or a form having both thereof,
a gene encoding a protein consisting of the amino acid sequence represented by SEQ ID NO: 3 in which one to several amino acids have been deleted, substituted or added and
a gene encoding a protein consisting of the amino acid sequence represented by SEQ ID NO: 4 in which one to several amino acids have been deleted, substituted or added.

In the gene of (7) above, the number of the amino acids which have been deleted, substituted or added is preferably one to 30, more preferably one to 25, further preferably one to 20, further preferably one to 15, further preferably one to 10, further preferably one to five, further preferably one to three.

The gene of (7) above is preferably a gene that encodes a protein consisting of the amino acid sequence represented by SEQ ID NO: 3 or 4 in which one to several amino acids have been deleted, substituted or added and that has a function of enhancing or conferring oxygen tolerance when the gene is introduced to a bacterium of the genus *Bifidobacterium*.

### [Culture under Aerobic Conditions]

The culture method of the invention includes a step of culturing the bacterium of the genus *Bifidobacterium* of this case under aerobic conditions.

The "aerobic conditions" refers to conditions under which an aerobic bacterium can respire aerobically. That is, the aerobic conditions include not only an aerobic environment in which the culture environment contains oxygen but also include an anaerobic environment in which the culture environment does not contain oxygen but in which the culture medium contains dissolved oxygen at a certain concentration.

In the culture method of the invention, examples of the aerobic conditions include conditions in which the oxygen concentration of the culture environment is preferably 1% or more, more preferably 3% or more, further preferably 6% or more.

The oxygen concentration of the culture environment can be, for example, 21% or less, preferably 18% or less, more preferably 12% or less.

In the culture method of the invention, culture of the related art which includes aeration with an anaerobic gas such as carbon dioxide gas is not required because the oxygen concentration of the culture environment is adjusted as described above. In the culture method of the invention, the bacterium of the genus *Bifidobacterium* is handled more easily than in a method of the related art.

The bacterium of the genus *Bifidobacterium* cultured by the culture method of the invention is easily handled in an aerobic environment and thus can be provided in a state having a high viable cell ratio.

Moreover, when the culture method of the invention is conducted in an anaerobic environment, dissolved oxygen in the culture medium may be adjusted to a certain concentration or more.

Here, the "culture environment" refers to the ambient atmosphere of the culture medium for culturing the bacterium of the genus *Bifidobacterium* of this case.

When the bacterium of the genus *Bifidobacterium* of this case is cultured in a liquid culture medium after the oxygen concentration in the culture environment is adjusted as described above, the bacterium may be cultured while the liquid culture medium is stirred.

Even when the efficiency of incorporation of the oxygen in the culture environment into the liquid culture medium is increased by stirring and when the oxygen concentration of the liquid culture medium is increased, the bacterium of the genus *Bifidobacterium* of this case is tolerant to oxygen and thus can be cultured without any problem. Furthermore, through culture with stirring, the nutrients in the liquid culture medium are supplied uniformly, and the growth efficiency of the bacterium of the genus *Bifidobacterium* of this case can be increased.

The bacterium of the genus *Bifidobacterium* of this case may be inoculated and cultured on a solid culture medium after the oxygen concentration in the culture environment is adjusted as described above.

In the culture method of the invention, culture of the related art which includes aeration with an anaerobic gas such as carbon dioxide gas is not required because the oxygen concentration of the culture environment is adjusted as described above. In the culture method of the invention, the bacterium of the genus *Bifidobacterium* is handled more easily than in solid culture of the related art.

The culture medium for culturing the bacterium of the genus *Bifidobacterium* of this case is not particularly limited, and a culture medium which is generally used for culturing a bacterium of the genus *Bifidobacterium* can be used. That is, as a carbon source, for example, saccharides such as glucose, galactose, lactose, arabinose, mannose, sucrose, fructose, cellobiose, trehalose, starch, a starch hydrolysate and molasses can be used depending on the assimilation properties. As a nitrogen source, for example, ammonia, and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, potassium sulfate and ferrous sulfate can be used. Furthermore, organic components such as peptone, soybean powder, a defatted soybean cake, meat extract and yeast extract may also be used.

The dissolved oxygen concentration of the culture medium can be 0.01 to 7 ppm, 0.01 to 3 ppm or 0.01 to 1 ppm.

In the culture method of the invention, culture of the related art which includes aeration with an anaerobic gas such as carbon dioxide gas is not required because the dissolved oxygen concentration of the culture medium is adjusted as described above. In the culture method of the invention, the bacterium of the genus *Bifidobacterium* is handled more easily than in a method of the related art.

The bacterium of the genus *Bifidobacterium* cultured by the culture method of the invention is easily handled in an aerobic environment and thus can be provided in a state having a high viable cell ratio.

The culture temperature may be 25 to 50°C and is preferably 35 to 42°C.

### <Modified Bacterium of Genus Bifidobacterium>

The invention also relates to a modified bacterium of the genus *Bifidobacterium* having enhanced or conferring oxygen tolerance obtained by introducing any one gene selected from (1) to (7) described above. The explanation in the item <Culture Method> above applies to the genes of (1) to (7).

A case in which the oxygen tolerance becomes stronger after the introduction of the gene of this case than that before the introduction applies to the "enhancement" of oxygen tolerance in the present specification. Moreover, a case in which the bacterium does not have oxygen tolerance before the introduction of the gene of this case but acquires oxygen tolerance through the introduction of the gene of this case applies to the "conferring" of oxygen tolerance in the present specification. Here, the degree of oxygen tolerance can be evaluated quantitatively with the viable cell ratio, the CFU or the like after culturing under aerobic conditions.

As the method for introducing the gene of this case into a bacterium of the genus *Bifidobacterium*, a known genetic engineering method can be used without any restriction. The competence method using the ability to incorporate DNA, the electroporation method using a high-voltage pulse and the like may be used. Moreover, when the gene of this case is to be incorporated into a chromosome of a bacterium of the genus *Bifidobacterium*, a method using homologous recombination or a site-specific incorporation method can be used.

The modified bacterium of the genus *Bifidobacterium* of the invention can be cultured even under anaerobic conditions and can also be cultured under aerobic conditions because the gene of this case has been introduced.

The modified bacterium of the genus *Bifidobacterium* of the invention has enhanced or conferred oxygen tolerance in addition to the original functions that the bacterium of the genus *Bifidobacterium* has and thus is provided as a bacterium of the genus *Bifidobacterium* that is strong against oxygen stress and that has excellent functionality.

The modified bacterium of the genus *Bifidobacterium* of the invention is easily handled in an aerobic environment and thus can be provided in a state having a high viable cell ratio.

### <Activated Bacterium of Genus Bifidobacterium>

Another aspect of the invention relates to an activated bacterium of the genus *Bifidobacterium* in which any one gene selected from (1) to (7) described above is upregulated. The explanation in the item <Culture Method> above applies to the genes of (1) to (7).

Here, the activated bacterium of *Bifidobacterium* is a bacterium of *Bifidobacterium* which has any one gene selected from (1) to (7) above and in which the gene is upregulated. In other words, the activated bacterium of the genus *Bifidobacterium* is a bacterium of the genus *Bifidobacterium* which has any one gene selected from (1) to (7) above and in which the expression level of any one gene selected from (1) to (7) above is increased.

When the bacterium of the genus *Bifidobacterium* having any one gene selected from (1) to (7) above is cultured under aerobic conditions, the gene can be upregulated. That is, whether the bacterium of the genus *Bifidobacterium* is activated can be examined by deciding the expression levels of the gene products (for example, mRNA and protein) of any one gene selected from (1) to (7) above after culturing under anaerobic conditions and aerobic conditions. Specifically, the expression levels of a gene when a bacterium of the genus Bifidobacterium having any one gene selected from (1) to (7) above is cultured under anaerobic conditions is compared with that when it is cultured under aerobic conditions. When the gene expression is increased through the aerobic culture, it can be found that the bacterium of the genus *Bifidobacterium* is the activated bacterium of the genus *Bifidobacterium* in the invention.

The gene related to oxygen tolerance of the activated bacterium of the genus *Bifidobacterium* of the invention is upregulated, and thus the bacterium is provided as a bacterium of the genus *Bifidobacterium* that is strong against oxygen stress and that has excellent functionality.

The activated bacterium of the genus *Bifidobacterium* of the invention is easily handled in an aerobic environment and thus can be provided in a state having a high viable cell ratio.

### <Production Method>

The invention relates to a method for producing a composition containing a bacterium of the genus *Bifidobacterium* including the culture method described above. That is, the production method of the invention is characterized by including a step of culturing the bacterium of the genus *Bifidobacterium* of this case under aerobic conditions. The production method of the invention may include a step which is required for producing a target composition in addition to the culture step.

In the production method of the invention, because a bacterium of the genus *Bifidobacterium* having excellent oxygen tolerance is used, not only the culturing step but also all the production steps can be conducted under aerobic conditions. Therefore, the production method of the invention does not require any step including aeration with an anaerobic gas such as carbon dioxide gas in all the production steps and is easily handled.

The composition obtained by the production method of the invention is easily handled in an aerobic environment and thus can be provided in a state having a high viable cell ratio of the bacterium of the genus *Bifidobacterium*.

In the production method of the invention, as the bacterium of the genus *Bifidobacterium* which is cultured under aerobic conditions, the bacterium of the genus *Bifidobacterium* of this case, the modified bacterium of the genus *Bifidobacterium* described above or the activated bacterium of the genus *Bifidobacterium* may be used. The specific aspects thereof are as explained in the items <Modified Bacterium of Genus *Bifidobacterium*> above and <Activated Bacterium of Genus *Bifidobacterium*> above.

The "composition" produced by the production method of the invention includes not only a form of a final product which is provided to a consumer, such as a food composition and a pharmaceutical composition, but also all of a culture of the bacterium of the genus *Bifidobacterium* of this case after the culture step, a form of an additive used for obtaining the final product and a form of an intermediate composition generated in the process of obtaining the final product.

The bacterium of the genus *Bifidobacterium* of this case contained in the composition produced by the production method of the invention may be a living bacterium or a dead bacterium. A preferable example of the form of the final product is a food composition or a pharmaceutical composition. The food composition or the pharmaceutical composition can be produced by adding living cells or dead cells of the bacterium of the genus *Bifidobacterium* of this case cultured by the culture method described above to a general raw material of a food or a medicine. The food composition or the pharmaceutical composition can be produced in the same manner as a general food or drink or a general medicine except that the bacterial cells are added. The bacterial cells may be added at any stage of the production step. When living cells of the bacterium of the genus *Bifidobacterium* of this case are used during the production step, the bacterium of the genus *Bifidobacterium* of this case contained in the composition can be formed into dead cells through sterilization treatment such as heating and pulverization at the end.

Because the composition produced by the production method of the invention contains a bacterium of *Bifidobacterium*, an effect of improving the intestinal microbiota and the like can be expected when an animal takes the composition. The intake of the composition may be use for a therapeutic purpose or use for a non-therapeutic purpose.

Here, the "non-therapeutic purpose" is a concept which does not include medical practice, namely treatment of a human body by therapy. Examples thereof include health promotion and beauty treatment.

The "improvement" means: improvement of a disease, a symptom or the conditions; prevention or delay of deterioration of a disease, a symptom or the conditions; or reversal, prevention or delay of progress of a disease or a symptom.

The "prevention" means prevention or delay of the onset of a disease or a symptom in the subject of the application or reduction of the risk of a disease or a symptom of the subject of the application.

When the composition produced by the production method of the invention is used for a non-therapeutic purpose, the composition can be administered to a healthy individual as the subject. The healthy individual may mean an individual having healthy intestinal microbiota or an individual without any disease caused by a disorder of the intestinal microbiota. Examples of the disease caused by a disorder of the intestinal microbiota include diarrhea and constipation. When the composition produced by the production method of the invention is used for a non-therapeutic purpose, the composition can be used for regulating the excellent intestinal microbiota of a healthy individual further better. Moreover, diarrhea or constipation of a healthy individual can be prevented.

When the composition produced by the production method of the invention is used for a therapeutic purpose, the composition can be administered to a non-healthy individual as the subject. An example of the non-healthy individual is an individual having a disease related to the intestinal microbiota. Such a disease is a bowel disease, and examples thereof include small intestinal tumor, gastrointestinal bleeding, intussusception, gastrointestinal stromal tumor, malabsorption syndrome, Behcet's disease, colorectal cancer, polyp, colonic diverticulitis, acute appendicitis, irritable bowel syndrome, ulcerative colitis, ischemic colitis and Crohn's disease inflammation. When the composition produced by the production method of the invention is used for a therapeutic purpose, the composition can regulate the intestines of a non-healthy individual, and improvement, prevention or treatment of diarrhea or constipation can become possible. When the oral composition for reaching an intestine of the invention is used for a therapeutic purpose, the composition can be used for improving the intestinal microbiota of a non-healthy individual.

The composition produced by the production method of the invention may be used for a human or a nonhuman animal (preferably a mammal) as the subject of application, preferably for a human or a pet, more preferably for a human. Furthermore, the subject to which the present technique is applied is not particularly limited as long as the subject wishes for a probiotic effect, and examples thereof include an infant, a small child, a child, an adult, a middle-aged individual, an aged individual, a healthy individual and those with poor enteric environment. Of these, the present technique is preferably used for an infant, a small child, an adult, an aged individual or those with poor enteric environment.

Regarding the administration or the intake of the composition produced by the production method of the invention, the composition is preferably taken continuously for at least one week and more preferably taken continuously for at least four weeks. During the administration or intake period, the composition is desirably taken every day.

The used amount of the composition produced by the production method of the invention is not particularly limited, but based on the dry weight of the bacterial cells, it is preferably 0.01 to 10000 mg/body weight kg/day, more preferably 0.01 to 1000 mg/body weight kg/day, still further preferably 0.1 to 100 mg/body weight kg/day.

Examples of the food composition include: drinks such as formula, soft drinks, carbonated drinks, nutritional drinks, fruit juices and lactic acid bacteria beverages (including concentrated undiluted solutions of the drinks and powders for preparation thereof); frozen desserts such as ice creams, sherbets and shaved ice; confectioneries such as sweets, chewing gums, candies, gums, chocolates, tablet candies, snacks, biscuits, jellies, jams, creams and baked sweets; dairy products such as processed milk, milk beverages, fermented milk, drinking yogurts and butter; breads; liquid foods such as enteral nutrition products and porridge, baby foods or sport drinks; and other functional foods. Moreover, the food composition may be a supplement and may be, for example, a supplement in the tablet form. In the case of a supplement, the bacterium of the genus *Bifidobacterium* of this case can be taken while the amount of meals and the calorie intake per day are not affected by other foods. Examples of the other commercial foods include baby foods, *Furikake* (dry Japanese seasonings) and seasonings for *Chazuke* (boiled rice with hot tea).

Here, the formulas include a powdered formula and a liquid formula. The powdered formula is defined by the Ministerial Ordinance Concerning Compositional Standards, etc. for Milk and Milk Products as "those obtained by processing raw milk, cow's milk, certified milk or a food produced therefrom as a raw material or using such a material as a major raw material, adding nutrients necessary for infants and small children and processing into powder".

The liquid formula is defined by the Ministerial Ordinance as "those obtained by processing raw milk, cow's milk, certified milk or a food produced therefrom as a raw material or using such a material as a major raw material, adding nutrients necessary for infants and small children and processing into liquid". The formulas also include those which contain a nutrient component such as proteins, oils and fats, carbohydrates, minerals and vitamins and which are processed into powder or liquid.

Moreover, the formulas further include "a powdered infant formula", "a liquid infant formula" and "a powdered formula for pregnant and parturient women and nursing mothers" of the food for special dietary uses provided by the Health Promotion Act, and also include aspects such as a powdered formula for infants and small children for infants of the age of 0 to 12 months, follow-up milk for infants and younger children of the age of 6 to 9 months or older (until the age of 3 years), a powdered formula for low birthweight infants for newborns with a body weight at birth less than 2500 g (low birthweight infants), various types of milk for treatment which are used for treating infants and small children with sickness such as milk allergy and lactose intolerance, a powdered formula for small children, nutritional powder for adults and nutritional powder for the aged.

The food composition can be used for either purpose of the therapeutic purpose and the non-therapeutic purpose, but is preferably used for the non-therapeutic purpose for a healthy individual as the subject.

The pharmaceutical composition may be for oral use. The dosage form of the pharmaceutical composition is not particularly limited, but in the case of oral administration, for example, the pharmaceutical composition can be formulated into a solid preparation such as powder, granules, tablets, troches and capsules or a liquid preparation such as a solution, a syrup, a suspension and an emulsion. In the case of parenteral administration, the pharmaceutical composition can be formulated into, for example, a suppository, a spray, an inhalant, ointment, patches or an injection. Moreover, for the formulation, an additive which is generally used as a carrier for formulation, such as excipients, binders, disintegrating agents, lubricants, stabilizers, flavoring agents, diluents, surfactants and solvents for injection, can be used. These components may be appropriately selected by one skilled in the art depending on the dosage form.

The pharmaceutical composition can be used for either purpose of the therapeutic purpose and the non-therapeutic purpose, but is preferably used for the therapeutic purpose for a test healthy individual as the subject.

When the food composition or the pharmaceutical composition is produced by the production method of the invention, the bacterial cell content of the bacterium of the genus *Bifidobacterium* of this case in the composition, in terms of the content taken per day, is preferably 1×10⁵ to 1×10¹² CFU/kg body weight/day, more preferably 1×10⁷ to 1×10¹¹ CFU/kg body weight/day, further preferably 1×10⁸ to 1×10¹⁰ CFU/kg body weight/day. Alternatively, the used amount per one individual (body weight) (in other words, dose) is preferably 10⁷ to 10¹⁴ CFU/day, more preferably 10⁸ to 10¹³ CFU/day, further preferably 10⁹ to 10¹² CFU/day. When the bacterium of *Bifidobacterium* is sterilized cells, the CFU can be replaced with the cells.

The composition produced by the production method of the invention preferably contains a prebiotic. Examples of the prebiotic include resistant dextrin, inulin, lactulose, galactooligosaccharides, fructooligosaccharides, soybean oligosaccharides, lactosucrose, xylooligosaccharides, isomaloligosaccharides, raffinose, coffee bean mannan oligosaccharides, gluconic acid, polydextrose, partially hydrolyzed guar gum, alginates, pectin, isomaltodextrin, barley-β glucane and human milk oligosaccharides (HMOs). Examples of the human milk oligosaccharides include 2'-fucosyllactose, 3'-sialyllactose, lacto-N-tetraose, 3-difucosyllactose, 3-fucosyllactose, 3-fucosyl-3'-sialyllactose, lacto-N-fucopentaose I, lacto-N-fucopentaose II, lacto-N-fucopentaose III, lacto-N-fucopentaose V, lacto-N-difucosylhexaose I, lacto-N-difucosylhexaose II, lacto-N-sialylpentaose, LSTa, LSTb and LSTc.

An embodiment of the production method of the invention includes a step of drying a bacterial culture containing the bacterium of the genus *Bifidobacterium* of this case which has been cultured by the culture method. According to the embodiment, a bacterial powder of the bacterium of the genus *Bifidobacterium* of this case can be obtained.

In the production method of the invention, because a bacterium of the genus *Bifidobacterium* having excellent oxygen tolerance is used, the step of drying can be conducted under aerobic conditions. By the production method of the invention, a composition which has a high viable cell ratio of the bacterium of the genus *Bifidobacterium* even after the step of drying can be provided.

The drying method is not particularly limited but may be a lyophilization method, a spray drying method or the like.

The bacterial cells of the bacterium of the genus *Bifidobacterium* of this case in the bacterial powder may be homogenized, and the homogenate may be one obtained by homogenizing a viable bacterium, one obtained by homogenizing a dead bacterium, or one subjected to heating, lyophilization or the like after homogenization. Of these, the culture of the bacterium of the genus *Bifidobacterium* of this case is preferably subjected to lyophilization from the viewpoint of increasing the viable cell ratio, and the culture is preferably subjected to spray drying from the viewpoint of increasing the production efficiency.

In an embodiment, a food composition is produced by adding the bacterial powder obtained by the drying step described above to a raw material composition. The food composition which can be produced in the embodiment is as described above. The raw material composition can be appropriately selected depending on the food composition as the aim of the production. The bacterial powder itself may be added to the raw material composition in an embodiment, and a solution obtained by dissolving the bacterial powder in water or the like may be added to the raw material composition in an embodiment.

In the production method of the invention, because the food composition is produced by adding the bacterial powder obtained by the drying step described above to the raw material composition, a food composition having a high viable cell ratio of the bacterium of the genus *Bifidobacterium* can be provided.

Moreover, the food composition may be produced through a fermentation step by the bacterium of the genus *Bifidobacterium* of this case added. Such a food composition may be a lactic acid bacteria beverage, a fermented milk or the like.

An embodiment includes a step of adding a starter culture containing the bacterium of the genus *Bifidobacterium* of this case which has been cultured by the culture method to a raw material composition and fermenting. The embodiment is effective when a lactic acid bacteria beverage or a fermented milk is produced, for example.

The starter culture may contain another bacterium in addition to the bacterium of the genus *Bifidobacterium* of this case. The other bacterium is preferably a lactic acid bacterium, and specific examples thereof include *Lactococcus lactis*, *Streptococcus thermophilus* and *Lactobacillus delbrueckii*. The subspecies is not particularly restricted, but examples of *Lactococcus lactis* include *Lactococcus lactis* subsp. *lactis* and *Lactococcus lactis* subsp. *cremoris*. Moreover, an example of *Lactobacillus delbrueckii* is *Lactobacillus delbrueckii* subsp. *bulgaricus*.

The composition produced by the production method of the invention may be provided as a product contained in a packaging container the inside of which is an aerobic environment. The bacterium of the genus *Bifidobacterium* of this case is easily handled in an aerobic environment, and thus a high viable cell ratio can be maintained even when the bacterium is contained and provided in a packaging container the inside of which is an aerobic environment.

### <Screening Method>

The invention also relates to a method for screening a bacterium of the genus *Bifidobacterium* having oxygen tolerance including a step of selecting a bacterium of the genus *Bifidobacterium* using any one gene selected from (1) to (7) described above as an indicator.

As described above, a bacterium of the genus *Bifidobacterium* having the gene of this case has oxygen tolerance which enables culture under aerobic conditions. In other words, a bacterium of the genus *Bifidobacterium* having the gene of this case can be decided to have oxygen tolerance.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene consisting of the base sequence represented by SEQ ID NO: 1 and
a gene consisting of the base sequence represented by SEQ ID NO: 2.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene having an identity of 90% or more with the base sequence represented by SEQ ID NO: 1 and
a gene having an identity of 90% or more with the base sequence represented by SEQ ID NO: 2.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene consisting of the base sequence represented by SEQ ID NO: 1 in which one to several bases have been deleted, substituted or added and
a gene consisting of the base sequence represented by SEQ ID NO: 2 in which one to several bases have been deleted, substituted or added.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene consisting of a base sequence of DNA which hybridizes to DNA consisting of a complementary sequence of the base sequence represented by SEQ ID NO: 1 under stringent conditions and
a gene consisting of a base sequence of DNA which hybridizes to DNA consisting of a complementary sequence of the base sequence represented by SEQ ID NO: 2 under stringent conditions.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene consisting of a degenerate variant of the base sequence represented by SEQ ID NO: 1 and
a gene consisting of a degenerate variant of the base sequence represented by SEQ ID NO: 2.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene encoding a protein consisting of the amino acid sequence represented by SEQ ID NO: 3 and
a gene encoding a protein consisting of the amino acid sequence represented by SEQ ID NO: 4.

The screening method of the invention may be an embodiment of deciding that the bacterium of the genus *Bifidobacterium* to be tested has oxygen tolerance when the bacterium has either one of the genes below or an embodiment of deciding that the bacterium has oxygen tolerance when the bacterium has both of the genes,
a gene encoding a protein consisting of the amino acid sequence represented by SEQ ID NO: 3 in which one to several amino acids have been deleted, substituted or added and
a gene encoding a protein consisting of the amino acid sequence represented by SEQ ID NO: 4 in which one to several amino acids have been deleted, substituted or added.

The explanation in the item <Culture Method> above applies to the genes of (1) to (7) described above used as an indicator of the screening method of the invention.

The method for deciding the presence or absence of the gene of this case for the screening method of the invention is not particularly restricted. The presence or absence of the gene of this case may be decided using the genome DNA as a subject, or the presence or absence of the gene of this case may be decided indirectly by measuring the presence or absence of the mRNA, which is the transcription product thereof, or the protein, which is the translation product thereof.

For example, decision may be made by designing primers which specifically hybridize to the base sequence region of the gene of this case and using the PCR method using the genome of the bacterium of the genus *Bifidobacterium* to be tested as a template.

Moreover, the presence or absence of the gene of this case may be decided indirectly by extracting mRNA from the bacterium of the genus *Bifidobacterium* to be tested and using RT-PCR or an array which can specifically detect the transcription product of the gene of this case.

Furthermore, the presence or absence of the gene of this case may be decided indirectly by a method using an antibody of the protein encoded by the gene of this case, such as Western blotting.

By the screening method of the invention, a bacterium of the genus *Bifidobacterium* having excellent oxygen tolerance can be easily selected by screening using the genes of (1) to (7) described above as an indicator.

### <Composition for Starter Culture>

The invention also relates to a composition for a starter culture containing a bacterium of the genus *Bifidobacterium* having any one gene selected from (1) to (7) described above. The explanation in the item <Culture Method> above applies to the genes of (1) to (7).

The composition for a starter culture of the invention can be obtained by inoculating and culturing the bacterium of the genus *Bifidobacterium* of this case in a culture medium.

In the invention related to the composition for a starter culture, a modified bacterium of the genus *Bifidobacterium* can be used as the bacterium of the genus *Bifidobacterium* of this case. That is, in another embodiment of the invention related to the composition for a starter culture, a modified bacterium of the genus *Bifidobacterium* having enhanced or conferred oxygen tolerance obtained by introducing any one gene selected from (1) to (7) described above is contained. The explanation in the item <Modified Bacterium of Genus *Bifidobacterium*> above applies to the modified bacterium of the genus *Bifidobacterium*.

In the invention related to the composition for a starter culture, an activated bacterium of the genus *Bifidobacterium* can be used as the bacterium of the genus *Bifidobacterium* of this case. That is, in another embodiment of the invention related to the composition for a starter culture, an activated bacterium of the genus *Bifidobacterium* in which any one gene selected from (1) to (7) described above is upregulated is contained. The explanation in the item <Activated Bacterium of Genus *Bifidobacterium*> above applies to the activated bacterium of the genus *Bifidobacterium*.

The composition for a starter culture of the invention can be in a form of a composition for a fermented milk starter. The composition for a fermented milk starter can be obtained by inoculating and culturing the bacterium of the genus *Bifidobacterium* of this case in a cow's milk medium containing whole milk, skim milk, reconstituted milk or the like or in a synthetic or semisynthetic culture medium containing lactose, glucose or the like as a main component.

When a fermented composition such as a fermented milk including drinking yogurt and an acidic milk beverage is produced using the composition for a starter culture of the invention, a fermented composition having a high viable bacterial count of the bacterium of the genus *Bifidobacterium* of this case can be obtained.

The composition for a starter culture of the invention can be in an embodiment containing a lactic acid bacterium in addition to the bacterium of the genus *Bifidobacterium* of this case. More specific contents of the embodiment are described in detail in the item <Composition> below.

### <Composition>

The invention also relates to a composition containing
a bacterium of the genus *Bifidobacterium* having any one gene selected from (1) to (7) described above and
one, two or more lactic acid bacteria selected from *Lactococcus lactis*, *Streptococcus thermophilus*, *Lactobacillus bulgaricus* and *Lactobacillus delbrueckii*.

The explanation in the item <Culture Method> above applies to the genes of (1) to (7).

In the invention related to the composition, a modified bacterium of the genus *Bifidobacterium* can be used as the bacterium of the genus *Bifidobacterium* of this case.

That is, in another embodiment of the invention related to the composition,
a modified bacterium of the genus *Bifidobacterium* having enhanced or conferred oxygen tolerance obtained by introducing any one gene selected from (1) to (7) described above and
one, two or more lactic acid bacteria selected from *Lactococcus lactis*, *Streptococcus thermophilus*, *Lactobacillus bulgaricus* and *Lactobacillus delbrueckii* are contained.

The explanation in the item <Modified Bacterium of Genus *Bifidobacterium*> above applies to the modified bacterium of the genus *Bifidobacterium*.

In the invention related to the composition, an activated bacterium of the genus *Bifidobacterium* can be used as the bacterium of the genus *Bifidobacterium* of this case.

That is, in another embodiment of the invention related to the composition,
an activated bacterium of the genus *Bifidobacterium* in which any one gene selected from (1) to (7) described above is upregulated and
one, two or more lactic acid bacteria selected from *Lactococcus lactis*, *Streptococcus thermophilus*, *Lactobacillus bulgaricus* and *Lactobacillus delbrueckii* are contained.

The explanation in the item <Activated Bacterium of Genus *Bifidobacterium*> above applies to the activated bacterium of the genus *Bifidobacterium*.

The bacterium of the genus *Bifidobacterium* of this case and the lactic acid bacteria described above have oxygen tolerance which enables culture even under aerobic conditions. That is, the composition of the invention can be considered as a mixture of *a Bifidobacterium* strain and a lactic acid bacterium which can be cultured even under aerobic conditions. Due to such a property, the composition of the invention is useful as a starter culture.

When the composition of the invention is used as a starter culture, a fermented composition having high viable cell ratios of the bacterium of the genus *Bifidobacterium* of this case and the lactic acid bacterium can be provided.

The composition of the invention can be in a form of a composition for a fermented milk starter. The composition for a fermented milk starter can be obtained by inoculating and culturing the bacterium of the genus *Bifidobacterium* of this case in a cow's milk medium containing whole milk, skim milk, reconstituted milk or the like or in a synthetic or semisynthetic culture medium containing lactose, glucose or the like as a main component.

When a fermented composition such as a fermented milk including drinking yogurt and an acidic milk beverage is produced using the composition of the invention as a fermented milk starter, a fermented composition having high viable bacterial counts of the bacterium of the genus *Bifidobacterium* of this case and the lactic acid bacterium can be obtained.

### Examples

### <Test Example>

### (1) Used Bacteria of Genus Bifidobacterium

In this test example, the eight bacterial strains of the genus *Bifidobacterium* below were used.
MCC02042 (Example 1): *Bifidobacterium longum* subsp. *infantis* specified by NITE BP-03068;
MCC10110 (Example 2): *Bifidobacterium longum* subsp. *infantis*. A bacterial strain stored in the Research & Information Center of MORINAGA MILK INDUSTRY CO., LTD. (Higashihara 5-1-83, Zama City, Kanagawa 252-8583)
JCM1210: *Bifidobacterium longum* subsp. *infantis* specified by JCM No. JCM1210;
JCM1222: *Bifidobacterium longum* subsp. *infantis* specified by JCM No. JCM1222;
JCM1260: *Bifidobacterium longum* subsp. *infantis* specified by JCM No. JCM1260;
JCM11344: *Bifidobacterium longum* subsp. *infantis* specified by JCM No. JCM11344;
JCM11345: *Bifidobacterium longum* subsp. *infantis* specified by JCM No. JCM11345;
JCM11347: *Bifidobacterium longum* subsp. *infantis* specified by JCM No. JCM11347;

### (2) Oxygen Tolerance Test

The bacterial strains (MCC2042, MCC10110, JCM1210, JCM1222, JCM1260, JCM11344, JCM11345 and JCM11347) were cultured in MRS culture medium (manufactured by BD Difco) at 37°C under anaerobic conditions for 16 hours. On BL agar plates (manufactured by EIKEN CHEMICAL CO., LTD.), 100 µL of the respective bacterial cultures after the culture were spread, and the bacterial strains were cultured at 37°C under aerobic conditions for 48 hours. The presence and absence of colony formation on the BL agar plates were evaluated. As a result, only MCC2042 and MCC10110 formed colonies, and the remaining bacterial strains did not form any colony. From the above results, it was decided that MCC2042 and MCC10110 were bacterial strains having oxygen tolerance.

### (3) Draft Genome Sequences

The bacterial strains (JCM1210, JCM1222, JCM1260, JCM11344, JCM11345 and JCM11347) were cultured in MRS culture medium at 37°C under anaerobic conditions for 16 hours. The culture solutions were centrifuged, and the bacterial cell pellets were obtained. DNA was extracted using DNeasy Blood & Tissue Kit (manufactured by QIAGEN N.V). The genome libraries were prepared using 1 ng of the extracted DNA and using Nextera XT DNA Sample Preparation Kit (manufactured by illumina, Inc.). After amplification by PCR (Thermo Fisher Scientific, Veriti200) and cleaning up, the size distributions of the tagged DNA fragments were analyzed using Agilent 2100 Bioanalyzer and High Sensitivity DNA Analysis Kit (manufactured by Agilent Technologies, Inc.).

The sequences of the libraries were analyzed using MiSeq Personal Sequencing System and MiSeq Reagent Kit v2 (500 cycles). The obtained sequence data were trimmed and assembled using CLC Genomics Workbench (manufactured by QIAGEN N.V). Default settings were used, but the contigs of 2000 bp or less were deleted.

### (4) Complete Genome Sequences

The bacterial strains (MCC2042 and MCC10110) were cultured in MRS culture medium at 37°C under anaerobic conditions for 16 hours. The culture solutions were centrifuged, and the bacterial cell pellets were obtained. DNA was extracted using NucleoBond high-molecular-weight (HMW) DNA kit (manufactured by MACHEREY-NAGEL GmbH & Co. KG). The genome libraries were prepared using SMRTbell Express Template Prep Kit (manufactured by Pacific Biosciences of California, Inc.). The size distributions were observed by pulsed-field electrophoresis, and the sizes were selected using BluePippi (manufactured by Sage Science, Inc.). The sequences were analyzed using PacBio RSII, which is a long read sequencer, and SMRT Cell 8 Pac V3. The obtained sequence data were assembled using canu. Default settings were used.

### (5) Comparative Genomics

The Fasta files of the obtained complete genomes and the draft genomes were used as the input files, and faa files were obtained through annotation using Bakta. All the obtained faa files were used as the input files, and ortholog tables were obtained using sonic paranoid2 (NPL 1). The designated parameters were --mmseqs 7.5 and --go. The ortholog groups which existed only in MCC2042 and MCC10110 and which did not exist in the other bacterial strains were extracted from the obtained ortholog tables, and a list of the amino acid sequences of the genes of MCC2042 included in the ortholog groups was obtained. The EC numbers (Enzyme Commission numbers) of these amino acid sequences were predicted using CLEAN (NPL 2).

As shown in (2) above, MCC2042 and MCC10110 could form colonies under aerobic conditions. It was believed that the two bacterial strains had higher ability of degrading oxygen than the other bacterial strains. That is, the genes responsible for the oxygen tolerance exhibited by the two bacterial strains were presumed to be genes encoding oxidoreductase (the enzyme group in which the first number of the EC number is 1, also referred to as "the enzyme group with EC numbers starting with 1" below).

Based on the presumption, the list of the amino acid sequences obtained by the comparative genomics described above and the results of the presumption of the EC numbers were studied, and genes consisting of the base sequence represented by SEQ ID NO: 1 or 2 were identified as genes which the other bacterial strains did not have but only MCC2042 and MCC10110 had and which encoded amino acid sequences of the enzyme group with EC numbers starting with 1 (oxidoreductase).

In this regard, of the genes which the other bacterial strains did not have but which only MCC2042 and MCC10110 had, the genes other than the genes consisting of the base sequence represented by SEQ ID NO: 1 or 2 were closely examined based on the estimated EC numbers, and it could not be reasonably concluded that the genes were possibly involved in the oxygen tolerance.

From the above results, it was concluded that the genes responsible for the oxygen tolerance observed only in MCC2042 and MCC10110 in the test in (2) above are the genes consisting of the base sequence represented by SEQ ID NO: 1 or 2.

### <Production Example 1>

*Bifidobacterium longum* subsp. *infantis* MCC02042 (NITE BP-03068) is added to 3 mL of an MRS liquid culture medium and cultured at 37°C for 24 hours under aerobic conditions, and then the culture solution is concentrated and lyophilized to obtain a lyophilized powder of the bacterium (bacterial powder). The bacterial powder, whey protein concentrate (WPC) and prebiotics (2'-FL and LNT) are mixed uniformly, and thus a composition is obtained. In 200 g of water, 20 g of the composition is dissolved, and thus a composition containing *Bifidobacterium longum* subsp. *infantis* MCC02042 (NITE BP-03068) is obtained.

### <Production Example 2>

Skim milk powder, cream and milk protein are mixed and dissolved to prepare 50 L of a milk raw material containing 3.0% (W/W) milk fat and 12.0% (W/W) solids-not-fat, and the milk raw material is heated to 70°C, homogenized at a pressure of 15 MPa, pasteurized at 90°C for 10 minutes and cooled to 40°C. In the pasteurized milk raw material, 500 mL of a culture of *Lactococcus lactis* subsp. *lactis*, 1.5×10¹⁴ CFU (colony forming unit) of frozen bacterial cells of *Bifidobacterium longum* subsp. *infantis* MCC02042 (NITE BP-03068) (manufactured by MORINAGA MILK INDUSTRY CO., LTD.) and 0.002% yogurt starter containing *Streptococcus thermophilus* and *Lactobacillus delbrueckii* subsp. *bulgaricus* are inoculated and cultured at 37°C for four hours, and thus a fermented milk is obtained. Hundred grams of the fermented milk are packed in a paper container having a volume of 120 mL (manufactured by SHINGI CORPORATION, having an opening of 71 mm and a height of 56 mm), and after the container is sealed with an aluminum foil lid, a plain-type fermented milk product is obtained by cooling to 10°C.

### <Production Example 3>

*Bifidobacterium longum* subsp. *infantis* MCC02042 (NITE BP-03068) is added to 3 mL of an MRS liquid culture medium and cultured at 37°C for 24 hours under aerobic conditions, and then the culture solution is concentrated and lyophilized to obtain a lyophilized powder of the bacterium (bacterial powder).

In 300 kg of warm water, 10 kg of desalted cow's milk whey protein powder (manufactured by MILEI GmbH), 6 kg of cow's milk casein powder (manufactured by Fonterra Cooperative Group Limited), 48 kg of lactose (manufactured by MILEI GmbH), 920 g of a mineral mixture (manufactured by Tomita Pharmaceutical Co., Ltd.), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) were dissolved and further heated and dissolved at 90°C for 10 minutes, and after adding 28 kg of modified fats (manufactured by Taiyo Yushi Corp.), the mixture was homogenized. Then, after pasteurization and concentration steps, the mixture was spray dried, and about 95 kg of a powdered formula was thus prepared. To the powdered formula, 100 g of the above lyophilized powder of *Bifidobacterium longum* subsp. *infantis* MCC02042 (NITE BP-03068) (1.8×10¹¹ cfu/g) triturated in starch is added, and thus about 95 kg of a *Bifidobacterium* strain·oligosaccharide-containing powdered formula is prepared. When the obtained powdered formula is dissolved in water and a formula solution having a total solid content concentration of 14% (w/V) as a standard formula concentration is obtained, 2.7×10⁹ cfu/100 mL can be obtained as the *Bifidobacterium* strain count in the formula solution.

### <Reference Example>

Using a temperature-sensitive plasmid which has been developed for gene modification of bifidobacteria, SEQ ID NOs: 1 and 2, which are the genes responsible for oxygen tolerance, are deleted based on NPL 3. Five hundred to thousand bases are selected from the upstream and downstream base sequences of each of the genes represented by SEQ ID NOs: 1 and 2 and amplified by PCR. As the primers, primers to which an overlapping sequence of 15 to 25 bases are added at the 5' end for joining the temperature-sensitive plasmid and the sequence to be amplified through In-Fusion reaction and for joining the upstream and downstream sequences to be amplified are used. The temperature-sensitive plasmid is treated with a restriction enzyme to linearize the plasmid. The linearized plasmid and the amplified base sequence are mixed and joined using In-Fusion Snap Assembly Master Mix (Takara Bio Inc.). The joined plasmid is transformed into DH5α (Takara Bio Inc.) and amplified. The amplified plasmid is extracted and introduced into *B. infantis* MCC02042 by the electroporation method. The *B. infantis* MCC02042 after the electroporation is cultured on a spectinomycin-containing GAM agar plate at 30°C. To cause a single crossover, the *B. infantis* MCC02042 transfected with the plasmid is cultured on a spectinomycin-containing Gifu anaerobic medium (GAM) agar plate at 39°C or higher and 42°C or lower. A formed colony is scraped and cultured in a culture medium obtained by adding 0.05% cysteine to the de Man, Rogosa, and Sharpe (MRS) for one to two days at 30°C. The bacterial culture is spread on an MRS agar plate and cultured at 37°C for one day. A colony is cloned and further cultured at 37°C. After the culture, a colony which has not grown is picked, and whether the SEQ ID NOs: 1 and 2, which are the oxygen tolerance genes, have been deleted is examined by PCR. When a strain is a deletion strain, the deletion strain is cultured in BL culture medium at 37°C for about 72 hours under aerobic conditions, and the difference in growth is compared with that of the wild-type strain (MCC2042) to evaluate that SEQ ID NOs: 1 and 2 are genes responsible for oxygen tolerance.

### Industrial Applicability

The invention can be applied to a production technique for a food or a medicine containing a bacterium of the genus *Bifidobacterium* having oxygen tolerance.

## Claims

1. A method for culturing a bacterium of the genus *Bifidobacterium* having any one gene selected from (1) to (7) below, comprising a step of culturing the bacterium of the genus *Bifidobacterium* under aerobic conditions:
(1) a gene consisting of a base sequence represented by SEQ ID NO: 1 or 2,
(2) a gene having an identity of 90% or more with the base sequence represented by SEQ ID NO: 1 or 2,
(3) a gene consisting of the base sequence represented by SEQ ID NO: 1 or 2 in which one to several bases have been deleted, substituted or added,
(4) a gene consisting of a base sequence of DNA which hybridizes to DNA consisting of a complementary sequence of the base sequence represented by SEQ ID NO: 1 or 2 under stringent conditions,
(5) a gene consisting of a degenerate variant of the base sequence represented by SEQ ID NO: 1 or 2,
(6) a gene encoding a protein consisting of an amino acid sequence represented by SEQ ID NO: 3 or 4, and
(7) a gene encoding a protein consisting of the amino acid sequence represented by SEQ ID NO: 3 or 4 in which one to several amino acids have been deleted, substituted or added.

2. The culture method according to claim 1, wherein the bacterium of the genus *Bifidobacterium* is *Bifidobacterium longum*.

3. The culture method according to claim 1, wherein the bacterium of the genus *Bifidobacterium* is *Bifidobacterium longum* subsp. *infantis*.

4. The culture method according to claim 1, wherein the bacterium of the genus *Bifidobacterium* is *Bifidobacterium longum* subsp. *infantis* MCC02042 (NITE BP-03068).

5. A method for producing a composition containing a bacterium of the genus *Bifidobacterium*, comprising the culture method according to any one of claims 1 to 4.

6. The production method according to claim 5, comprising a step of drying a bacterial culture containing the bacterium of the genus *Bifidobacterium* cultured by the culture method, wherein the composition is a bacterial powder obtained by the drying or a food composition containing the bacterial powder.

7. The production method according to claim 5, comprising a step of adding a starter culture containing the bacterium of the genus *Bifidobacterium* which has been cultured by the culture method to a raw material composition and fermenting.

8. A modified bacterium of the genus *Bifidobacterium* having enhanced or conferred oxygen tolerance obtained by introducing any one gene selected from (1) to (7) below:
(1) a gene consisting of a base sequence represented by SEQ ID NO: 1 or 2,
(2) a gene having an identity of 90% or more with the base sequence represented by SEQ ID NO: 1 or 2,
(3) a gene consisting of the base sequence represented by SEQ ID NO: 1 or 2 in which one to several bases have been deleted, substituted or added,
(4) a gene consisting of a base sequence of DNA which hybridizes to DNA consisting of a complementary sequence of the base sequence represented by SEQ ID NO: 1 or 2 under stringent conditions,
(5) a gene consisting of a degenerate variant of the base sequence represented by SEQ ID NO: 1 or 2,
(6) a gene encoding a protein consisting of an amino acid sequence represented by SEQ ID NO: 3 or 4, and
(7) a gene encoding a protein consisting of the amino acid sequence represented by SEQ ID NO: 3 or 4 in which one to several amino acids have been deleted, substituted or added.

9. A method for screening a bacterium of the genus *Bifidobacterium* having oxygen tolerance, comprising a step of selecting a bacterium of the genus *Bifidobacterium* using any one gene selected from (1) to (7) below as an indicator:
(1) a gene consisting of a base sequence represented by SEQ ID NO: 1 or 2,
(2) a gene having an identity of 90% or more with the base sequence represented by SEQ ID NO: 1 or 2,
(3) a gene consisting of the base sequence represented by SEQ ID NO: 1 or 2 in which one to several bases have been deleted, substituted or added,
(4) a gene consisting of a base sequence of DNA which hybridizes to DNA consisting of a complementary sequence of the base sequence represented by SEQ ID NO: 1 or 2 under stringent conditions,
(5) a gene consisting of a degenerate variant of the base sequence represented by SEQ ID NO: 1 or 2,
(6) a gene encoding a protein consisting of an amino acid sequence represented by SEQ ID NO: 3 or 4, and
(7) a gene encoding a protein consisting of the amino acid sequence represented by SEQ ID NO: 3 or 4 in which one to several amino acids have been deleted, substituted or added.

10. A composition for a starter culture, comprising a bacterium of the genus *Bifidobacterium* having any one gene selected from (1) to (7) below:
(1) a gene consisting of a base sequence represented by SEQ ID NO: 1 or 2,
(2) a gene having an identity of 90% or more with the base sequence represented by SEQ ID NO: 1 or 2,
(3) a gene consisting of the base sequence represented by SEQ ID NO: 1 or 2 in which one to several bases have been deleted, substituted or added,
(4) a gene consisting of a base sequence of DNA which hybridizes to DNA consisting of a complementary sequence of the base sequence represented by SEQ ID NO: 1 or 2 under stringent conditions,
(5) a gene consisting of a degenerate variant of the base sequence represented by SEQ ID NO: 1 or 2,
(6) a gene encoding a protein consisting of an amino acid sequence represented by SEQ ID NO: 3 or 4, and
(7) a gene encoding a protein consisting of the amino acid sequence represented by SEQ ID NO: 3 or 4 in which one to several amino acids have been deleted, substituted or added.

11. A composition, comprising:
a bacterium of the genus *Bifidobacterium* having any one gene selected from (1) to (7) below,
(1) a gene consisting of a base sequence represented by SEQ ID NO: 1 or 2,
(2) a gene having an identity of 90% or more with the base sequence represented by SEQ ID NO: 1 or 2,
(3) a gene consisting of the base sequence represented by SEQ ID NO: 1 or 2 in which one to several bases have been deleted, substituted or added,
(4) a gene consisting of a base sequence of DNA which hybridizes to DNA consisting of a complementary sequence of the base sequence represented by SEQ ID NO: 1 or 2 under stringent conditions,
(5) a gene consisting of a degenerate variant of the base sequence represented by SEQ ID NO: 1 or 2,
(6) a gene encoding a protein consisting of an amino acid sequence represented by SEQ ID NO: 3 or 4, and
(7) a gene encoding a protein consisting of the amino acid sequence represented by SEQ ID NO: 3 or 4 in which one to several amino acids have been deleted, substituted or added; and
one, two or more lactic acid bacteria selected from *Lactococcus lactis*, *Streptococcus thermophilus*, *Lactobacillus bulgaricus* an*d Lactobacillus delbrueckii*.
